# NEW EUROPEAN PATENT SPECIFICATION

(11) **EP 0 747 050 B2**
(45) Date of publication and mention of the opposition decision: **13.02.2013**
(45) Mention of the grant of the patent: 03.09.2003
(21) Application number: 96304291.6
(22) Date of filing: 07.06.1996
(51) Int. Cl.: A61K 31/415, A61K 9/00, A61K 9/20

(54) **Pharmaceutical compositions containing irbesartan**
Irbesartanhaltiges Arzneimittel
Compositions pharmaceutiques d'irbésartan

(30) Priority: 07.06.1995 US 472618
(43) Date of publication of application: 11.12.1996
(60) Divisional application: 02016237.6
(73) Proprietor: SANOFI, 75008 Paris (FR)
(72) Inventor: Ku, Cathy C., Martinsville, NJ 08836 (US); Sprockel, Omar L., Bridgewater, NJ 08807 (US); Rubitski, Beth A., Bedminster, NJ 07921 (US); Desai, Divyakant S., Robbinsville, NJ 08691 (US)
(74) Representative: Thouret-Lemaitre, Elisabeth

(56) References cited:
- EP-A- 0 475 898
- EP-A- 0 629 408
- EP-A- 0 708 103
- WO-A-91/14679
- US-A- 5 270 317
- US-A- 5 541 209
- 'Rote Liste 1994'

## Description

The present invention relates to pharmaceutical compositions containing irbesartan, in the form of a tablet as defined in claims 1-3.

Irbesartan, 2-n-butyl-4-spirocyclopentane-1-[(2'-(tetrazol-5-yl)biphenyl-4-yl)methyl]-2-imidazolin-5-one, is a potent, long-acting angiotensin II receptor antagonist which is particularly useful in the treatment of cardiovascular ailments such as hypertension and heart failure. Irbesartan has the following structure: 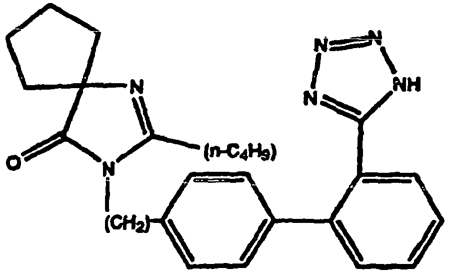 and is described in Bernhart et al., U.S. Patent No. 5,270,317, incorporated herein by reference. Preferred pharmaceutical compositions of this drug contain, as active ingredient(s), irbesartan alone or in combination with a diuretic such as hydrochlorothiazide.

Irbesartan may be administered in dosages containing a substantial quantity of the active agent (e.g., 75 - 300 mg). Certain physical properties of the drug present a challenge in developing formulations suitable for preparing a tablet having both a substantial quantity of active agent and a small enough tablet mass to allow ease of swallowing.

Irbesartan is, for example, a fluffy material, with relatively low bulk and tap densities. These properties make it difficult to formulate a large amount of the drug into a small tablet with uniformity of weight, hardness, and other desirable tablet properties. In addition, irbesartan has certain undesirable flow characteristics, for example, is sticky and can adhere to surfaces such as tablet punch faces and dies, causing problems in tableting, especially on a high speed tablet press. The low aqueous solubility of irbesartan also presents a challenge, since, to keep the tablet mass small, only limited amounts of excipients may be added to facilitate wetting, disintegration, and ultimately, rapid and complete drug release.

Thus, there is a need in the art for pharmaceutical compositions containing irbesartan, which have good properties for tablet formation, and yet which contain a low mass of excipients so that small, easily swallowed tablets with a high content of active agent may be prepared.

The present invention provides pharmaceutical compositions containing irbesartan, that are defined in claims 1-3 and which (1) have a minimal mass of added excipients, thereby allowing preparation of small, easily swallowed tablets which enhance patient acceptance and compliance, and yet which (2) have excellent properties for tablet formation, and (3) provide tablets with excellent wetting, disintegration, and ultimately, rapid and complete drug release properties.

A tablet formed from said composition has a dissolution performance such that about 80% or greater, preferably 85% or greater, of the irbesartan or salts thereof contained in said tablet dissolve within 30 minutes.

Said compositions can be employed in the reproducible manufacture of tablets on a large scale. The present compositions can, for example, be compressed on high speed tableting equipment (especially, a high speed tablet press) to form tablets which are uniform in both weight and content and which exhibit desirable physical properties, including elegant appearance, low friability, and fast disintegration time. Tablets prepared from the present compositions are capable of releasing the active component(s), by dissolution, in a fast and reproducible manner.

### Compositions

The following table recite compositions of the present invention which produce tablets of especially high quality and superior performance. Table A recites compositions containing irbesartan.

**TABLE A**

| IRBESARTAN | | |
|---|---|---|
| Preferred Ingredient | Component | Concentration Range (% w/w) |
| irbesartan | active drug | 20 - 50 |
| lactose hydrous | diluent | 1 - 70 |
| microcrystalline cellulose (e.g., Avicel® PH 102⁺) | diluent | 5-20 |
| pregelatinized starch (e.g., Starch® 1500⁺) | binder | 10 - 20 |
| croscarmellose sodium (e.g., Ac-Di-sol®⁺) | disintegrant | 4 - 8 |
| poloxamer, especifically poloxamer 188 (e.g., Pluronic® F68⁺) | surfactant | 1 - 6 |
| silicon dioxide (e.g., Syloid® 244⁺) | antiadherent | 0.25 - 5.0 (0.25 to 1.5 or, especially, 2.5 to 3.0) |
| magnesium stearate | lubricant | 0.5 - 1.5 |
| TOTAL | | 100 |

| | | |
|---|---|---|
| + These exemplary compounds may be used as desired throughout this specification as appropriate. For example, Starch® 1500 may be used as desired wherever pregelatinized starch appears in this specification. | | |

In the above compositions of Table A, the combination of magnesium stearate and silicon dioxide provides a superior lubrication effect while minimizing any decline in tablet dissolution performance; the intragranular:extragranular placement ratio of the disintegrant croscarmellose sodium is superior (e.g., 1:1); and the poloxamer surfactant improves the aqueous granulation of irbesartan (which is hydrophobic), eases the ejection of tablets after compression and accelerates the dissolution of the irbesartan active agent.

The present invention is described in further detail as follows. Pharmaceutical compositions according to the present invention are described in claims 1 to 3. The components employed in the compositions of the present invention should be pharmaceutically acceptable, particularly as described in the National Formulary (NF) or United States Pharmacopeia (USP).

The "dissolution performance" of a tablet, as used herein with respect to irbesartan, refers to the weight % of irbesartan, based on the total weight of irbesartan contained in the tablet, which dissolves within 30 minutes under the following conditions: using a tablet having a total weight of from 150 to 600 mg and a USP Apparatus 2, placing the tablet in 1000 mL of 0.1 N hydrochloric acid at 37°C, with a paddle speed of 50 rpm, and measuring the amount of irbesartan dissolved (especially, using UV at 244 nm) at 30 minutes. (If desired, the progress of dissolution may also be monitored at various time points.)

The "diluent" employed in a composition of the present invention is lactose hydrous and microcrystalline cellulose, in the range of 1 to 70% by weight of the composition. These compounds are capable of providing bulk to obtain a desired tablet mass. It is desirable to employ the diluent in an amount at the lower end of the weight range for the diluent.

The "binder" employed in a composition of the present invention is pregelatinized starch, in the range of 10 to 20% by weight of the composition. This compound is capable of facilitating granulation of the irbesartan into larger, denser, and/or more free-flowing particles.

The "disintegrant" employed in a composition of the present invention is croscarmellose sodium (cross-linked polymer of carboxymethyl-cellulose sodium), in the range of 4 to 8% by weight of the composition. This compound is capable of facilitating the break up of a tablet prepared from the composition when placed in contact with an aqueous medium.

The "antiadherent" employed in a composition of the present invention is silicon dioxyde, in the range of 0,25 to 5% by weight of the composition. This compound is capable of reducing the stickiness of the formulation, for example, preventing adherence to metal surfaces.

The "lubricant" employed in a composition of the present invention is magnesium stearate, in the range of 0,5 to 1,5% by weight of the composition. This compound is capable of preventing tableting problems, such as those relating to the release of a tablet prepared from the composition from the apparatus on which it is formed, for example, preventing adherence to the face of the upper punch (picking) or lower punch (sticking) of a tableting apparatus.

The "surfactant" employed in a composition of the present invention, is poloxamer 188, in the range of 1 to 6% by weight of the composition. This compound is capable of improving the wetting of the tablets and/or enhancing dissolution.

The present compositions preferably consist essentially of, most preferably, consist of the above-described components.

### Methods of Manufacture

Tablets may be prepared from the present compositions by any suitable method for forming tablets. Preferably, tablets are prepared from the present compositions by a wet granulation process. An exemplary such method comprises the following steps:
(a) preparing an intragranular composition by:
   (i) mixing the irbesartan, a portion of the diluent (preferably, from about 5 to about 80% by weight of the total diluent), a portion of the disintegrant (preferably, from about 50 to about 80% by weight of the total disintegrant), the binder, and, optionally, a portion of the antiadherent (preferably, from about 50 to about 80% by weight of the total antiadherent), to form a powder blend and, optionally, sizing the blend (e.g., milling the blend to break up aggregates);
   (ii) re-mixing the blend;
   (iii) granulating the blend with a granulating fluid, preferably water and/or an aqueous solution of the surfactant, to form granules (e.g., using a high shear mixer/granulator):
   (iv) drying the granules (e.g., in an oven or, preferably, in a fluid bed dryer); and
   (v) sizing the dried granules (e.g., by milling or screening);
(b) preparing a mixture of the sized granules of step (a) (v) with an extragranular composition by:
   (i) mixing the remainder of the diluent, the remainder of the disintegrant, the antiadherent or the remainder of the antiadherent, where one or more of these may be pre-blended, sized (e.g., milled to break up aggregates) and re-mixed prior to this step, with the sized granules from step (a)(v) to form a granule blend; and
   (ii) mixing the lubricant with the granule blend; and
(c) compressing the mixture from step (b) (ii) to form tablets (for example, employing a tablet press).

The solid starting materials of the present compositions are preferably screened prior to use. The weight ratio of water (preferably, purified water, USP or water for injection, USP) to solids employed in step (a)(iii) is preferably within the range of from about 0.25:1 to about 0.6:1.

The tablets may, optionally, be finished or coated such as by methods known in the art.

The tablets prepared from the compositions of the present invention preferably contain (per tablet) from about 25 to about 300 mg of irbesartan, most preferably from about 75 to 300 mg of irbesartan. The total weight of the tablets prepared is preferably from about 50 to about 600 mg. In addition to tablets, the compositions of the present invention may be used to prepare beads, granules for dispersion or capsules, the latter, for example, filled with powder or the aforementioned beads or granules. Methods such as those well known in the art may be used to prepare these dosage forms.

The compositions and tablets of the present invention may be used to treat or prevent disorders such as those described in U.S. Patent No. 5,270,317, incorporated herein by reference. Such disorders include cardiovascular disorders, for example, hypertension or heart failure, venous insufficiency, as well as glaucoma, diabetic retinopathy, renal insufficiency and various complaints of the central nervous system. The present compositions or tablets are preferably administered orally, in an effective amount, to a mammalian (especially, human) subject to treat or prevent the aforementioned disorders. For human subjects, preferred dosages of from about 75 mg to about 300 mg of irbesartan may be administered, for example, 1 to 2 times per day.

The following Examples are provided to illustrate the invention.

### EXAMPLE 1

### PREPARATION OF TABLETS CONTAINING IRBESARTAN

Tablets containing irbesartan were prepared in three potencies from the composition of the present invention described in the following Table 1: (1) 75 mg irbesartan with a total weight of 150 mg per tablet; (2) 150 mg irbesartan with a total weight of 300 mg per tablet; and (3) 300 mg irbesartan with a total weight of 600 mg per tablet.

**TABLE 1**

| Ingredient | Component | Concentration (% w/w) |
|---|---|---|
| **INTRAGRANULAR** | | |
| irbesartan | active drug | 50 |
| lactose hydrous, NF | diluent | 10.25 |
| pregelatinized starch, NF | binder | 15.0 |
| croscarmellose sodium, NF | disintegrant | 2.5 |
| poloxamer 188, NF | surfactant | 3.0 |

| **EXTRAGRANULAR** | | |
|---|---|---|
| microcrystalline cellulose, NF | diluent | 15.0 |
| croscarmellose sodium, NF | disintegrant | 2.5 |
| silicon dioxide, NF | antiadherent | 0.75 |
| magnesium stearate, USP | lubricant | 1.0 |
| **TOTAL** | 100.00 | 100.00 |

Using the above formulation, the tablets were prepared by a wet granulation process as follows. In this process, the total amount of water employed (by weight) was up to 50% of the total solids weight.

The irbesartan, lactose, pregelatinized starch, and a portion (1/2) of the croscarmellose sodium were mixed in a mixer. The powder blend prepared was passed through sizing equipment (cone mill or oscillator), and mixed in a mixer. The poloxamer 188 was dissolved in water (purified, USP or water for injection, USP) (25% of the weight of total solids), and used to wet granulate (with the further addition of water in an amount which was up to 25% of the weight of total solids as needed) the mixed powder. The granules obtained were dried (tray or fluid bed dryer) until the loss-on-drying (LOD) was 2% or less. The dried granules were passed through a screen or milled to obtain the proper size (1 to 3 mm).

The sized granules were mixed with the silicon dioxide, the microcrystalline cellulose and the remaining croscarmellose sodium in a mixer. The blend obtained was then mixed with the magnesium stearate. By compressing the mixture using tableting equipment, tablets were prepared for each potency having the compositions indicated in the following Table 2.

**TABLE 2**

| Ingredient | 75 mg Potency (mg) | 150 mg Potency (mg) | 300 mg Potency (mg) |
|---|---|---|---|
| irbesartan | 75.00 | 150.00 | 300.00 |
| lactose hydrous, NF | 15.38 | 30.75 | 61.50 |
| microcrystalline cellulose, NF | 22.50 | 45.00 | 90.00 |
| pregelatinized starch, NP | 22.50 | 45.00 | 90.00 |
| croscarmellose sodium, | 7.50 | 15.00 | 30.00 |
| poloxamer 188, NF (or Pluronic F68, NF) | 4.50 | 9.00 | 18.00 |
| silicon dioxide, NF | 1.12 | 2.25 | 4.50 |
| magnesium stearate, USP | 1.50 | 3.00 | 6.00 |
| Tablet Weight | 150.00 | 300.00 | 600.00 |

### EXAMPLE 2

### PREPARATION OF TABLETS CONTAINING IRBESARTAN

Tablets containing irbesartan were prepared in three potencies from the composition of the present invention described in the following Table 3: (1) 75 mg irbesartan with a total weight of 150 mg per tablet; (2) 150 mg irbesartan with a total weight of 300 mg per tablet; and (3) 300 mg irbesartan with a total weight of 600 mg per tablet.

**TABLE 3**

| Ingredient | Component | Concentration (% w/w) |
|---|---|---|
| **INTRAGRANULAR** | | |
| irbesartan | active drug | 50 |
| lactose hydrous, NF | diluent | 10.25 |
| pregelatinized starch, NF | binder | 15.0 |
| croscarmellose sodium, | disintegrant | 2.5 |
| NF | surfactant | 3.0 |
| silicon dioxide, NF | antiadherent | 2.0 |

| **EXTRAGRANULAR** | | |
|---|---|---|
| microcrystalline cellulose, NF | diluent | 13.0 |
| croscarmellose sodium, NF | disintegrant | 2.5 |
| silicon dioxide, NP | antiadherent | 0.75 |
| magnesium stearate, USP | lubricant | 1.0 |
| **TOTAL** | 100.00 | 100.00 |

Using the above formulation, the tablets were prepared by a wet granulation process as follows. In this process, the total amount of water employed (by weight) was up to 50% of the total solids weight.

The irbesartan, lactose, pregelatinized starch, a portion (1/2) of the croscarmellose sodium, and a portion (about 73%) of the silicon dioxide were mixed in a mixer. The powder blend prepared was passed through sizing equipment (cone mill or oscillator), and mixed in a mixer. The poloxamer 188 was dissolved in water (purified, USP or water for injection, USP) (25% of the weight of total solids), and used to wet granulate (with the further addition of water in an amount which was up to 25% of the weight of total solids as needed) the mixed powder. The granules obtained were dried (tray or fluid bed dryer) until the loss-on-drying (LOD) was 2% or less. The dried granules were passed through a screen or milled to obtain the proper size (1 to 3 mm).

The sized granules were mixed with the remaining silicon dioxide, the microcrystalline cellulose and the remaining croscarmellose sodium in a mixer. The blend obtained was then mixed with the magnesium stearate. By compressing the mixture using tableting equipment, tablets were prepared for each potency having the compositions indicated in the following Table 4.

**TABLE 4**

| Ingredient | 75 mg Potency (mg) | 150 mg Potency (mg) | 300 mg Potency (mg) |
|---|---|---|---|
| irbesartan | 75.00 | 150.00 | 300.00 |
| lactose hydroys, NF | 15.50 | 30.75 | 61.50 |
| Microcrystalline cellulose, NF | 19.50 | 39.00 | 78.50 |
| pregelatinized starch, NF | 22.50 | 45.00 | 90.00 |
| croscarmellose sodium, NP | 7.50 | 15.00 | 30.00 |
| poloxamer 188, NF (or Pluronic F68, NF) | 4.50 | 9.00 | 18.00 |
| silicon dioxide, NF | 4.12 | 8.25 | 16.50 |
| magnesium stearte, USP | 1.50 | 3.00 | 6.00 |
| Tablet Weight | 150.00 | 300.00 | 600.00 |

Tablets comprising irbesartan or a pharmaceutically acceptable salt thereof, prepared (such as is described herein) by mixing an extragranular composition with granules comprising an antiadherent (silicon dioxide), may dissolve more rapidly and/or completely, and thus may exhibit an improved dissolution performance.

## Claims

1. A pharmaceutical composition in the form of a tablet, wherein said composition comprises, based on weight:
(a) from 20 to 50% irbesartan
(b) from 1 to 70% diluent, wherein said diluent is lactose hydrous and microcrystalline cellulose;
(c) from 10 to 20% binder, wherein said binder is pregelatinized starch;
(d) from 4 to 8% disintegrant, wherein said disintegrant is croscarmellose sodium;
(e) from 0,25% to 5% antiadherent, wherein said antiadherent is silicon dioxide;
(f) from 0,5 to 1,5% lubricant, wherein said lubricant is magnesium stearate;
(g) from 1 to 6% surfactant, wherein the surfactant is poloxamer 188,
wherein the tablet formed from said composition has a dissolution performance such that 80% or greater of the irbesartan or salt thereof contained in said tablet dissolves within 30 minutes, wherein the dissolution performance is measured using a tablet having a total weight of from 150 to 600 mg and USP apparatus 2, placing the tablet in 1000 mL of 0.1N hydrochloric acid at 37°C with a paddle speed of 50 rpm and measuring the irbesartan dissolved at 30 minutes.

2. The pharmaceutical composition according to claim 1 comprising, based on weight, 50 % irbesartan ; 10.25 % lactose hydrous ; 15.0 % pregelatinized starch ; 5.0 % croscarmellose sodium ; 3.0 % poloxamer 188 ; 15 % microcrystalline cellulose ; 0.75 % silicon dioxide ; and 1.0 % magnesium stearate.

3. The pharmaceutical composition according to claim 1 comprising, based on weight, 50 % irbesartan ; 10.25 % lactose hydrous 15.0 % pregelatinized starch ; 5.0 % croscarmellose sodium ; 3.0 % poloxamer 188 ; 13 % microcrystalline cellulose ; 2.75 % silicon dioxide ; and 1.0 % magnesium stearate.

## Patentansprüche

1. Pharmazeutische Zubereitung in Form einer Tablette, umfassend, bezogen auf das Gewicht:
(a) 20 bis 50 % Irbesartan;
(b) 1 bis 70 % Verdünnungsmittel, wobei es sich bei dem Verdünnungsmittel um wasserhaltige Lactose und mikrokristalline Cellulose handelt;
(c) 10 bis 20 % Bindemittel, wobei es sich bei dem Bindemittel um vorgelatinierte Stärke handelt;
(d) 4 bis 8 % Sprengmittel, wobei es sich bei dem Sprengmittel um Natriumcroscarmellose handelt;
(e) 0,25 bis 5 Antihaftmittel, wobei es sich bei dem Antihaftmittel um Siliciumdioxid handelt;
(f) 0,5 bis 1,5 % Gleitmittel, wobei es sich bei dem Gleitmittel um Magnesiumstearat handelt;
(g) 1 bis 6 % oberflächenaktives Mittel, wobei es sich bei dem oberflächenaktiven Mittel um Poloxamer 188 handelt,
wobei die aus dieser Zubereitung geformte Tablette ein solches Lösungsverhalten zeigt, daß 80 % oder mehr des in der Tablette vorhandenen Irbesartans oder des Salzes davon sich innerhalb von 30 Minuten lösen, wobei das Lösungsverhalten mit einer Tablette mit einem Gesamtgewicht von 150 bis 600 mg und USP-Apparat 2 gemessen wird, indem man die Tablette bei 37°C mit einer Paddelgeschwindigkeit von 50 U/min in 1000 ml 0,1 N Salzsäure gibt und nach 30 Minuten das gelöste Irbesatan misst.

2. Pharmazeutische Zubereitung nach Anspruch 1, umfassend, bezogen auf das Gewicht, 50 % Irbesartan; 10,25 % wasserhaltige Lactose; 15,0 % vorgelatinierte Stärke; 5,0 % Natriumcroscarmellose; 3,0 % Poloxamer 188; 15 % mikrokristalline Cellulose; 0,75 % Siliciumdioxid; und 1,0 % Magnesiumstearat.

3. Pharmazeutische Zubereitung nach Anspruch 1, umfassend, bezogen auf das Gewicht, 50 % Irbesartan; 10,25 % wasserhaltige Lactose; 15,0 % vorgelatinierte Stärke; 5,0 % Natriumcroscarmellose; 3,0 % Poloxamer 188; 13 % mikrokristalline Cellulose; 2,75 % Siliciumdioxid; und 1,0 % Magnesiumstearat.

## Revendications

1. Composition pharmaceutique sous forme de comprimé, ladite composition comprenant, en poids :
(a) de 20 à 50% d'irbesartan ;
(b) de 1 à 70% d'un diluant, ledit diluant étant du lactose hydraté et de la cellulose microcristalline ;
(c) de 10 à 20% d'un agent liant, ledit liant étant de l'amidon prégélifié ;
(d) de 4 à 8% d'un agent désintégrant, ledit désintégrant étant de la croscarmellose sodique ;
(e) de 0,25 à 5% d'un agent anti-adhérent, ledit anti-adhérent étant du dioxyde de silicium ;
(f) de 0,5 à 1,5% d'un agent lubrifiant, ledit lubrifiant étant du stéarate de magnésium ;
(g) de 1 à 6% d'un agent tensioactif, ledit agent tensio-actif étant du poloxamer 188,
Le comprimé formé à partir de ladite composition ayant une capacité de dissolution telle que 80% ou plus de l'irbesartan ou du sel de ce composé contenu dans ledit comprimé se dissout en 30 minutes, la capacité de dissolution étant mesurée à l'aide d'un comprimé ayant un poids total de 150 à 600mg et d'un appareil USP 2 en plaçant le comprimé dans 1000ml d'acide chlorhydrique o,IN at 37°C avec une vitesse des pales de 50 t/min et en mesurant l'irbesartan dissous à 30 minutes.

2. Composition pharmaceutique selon la revendication 1, comprenant en poids, 50% d'irbesartan ; 10,25% de lactose hydraté ; 15,0% d'amidon prégélifié ; 5,0% de croscarmellose sodique ; 3,0% de poloxamer 188 ; 15% de cellulose microcristalline ; 0,75% de dioxyde de silicium ; et 1,0% de stéarate de magnésium.

3. Composition pharmaceutique selon la revendication 1, comprenant en poids, 50% d'irbesartan ; 10,25% de lactose hydraté ; 15,0% d'amidon prégélifié ; 5,0% de croscarmellose sodique ; 3,0% de poloxamer 188 ; 13% de cellulose microcristalline ; 2,75% de dioxyde de silicium ; et 1,0% de stéarate de magnésium.
